# EUROPEAN PATENT SPECIFICATION

(11) **EP 4 480 440 B1**
(45) Date of publication and mention of the grant of the patent: **15.04.2026**
(21) Application number: 22928431.0
(22) Date of filing: 21.12.2022
(51) Int. Cl.: A61B 34/35, A61M 25/082, A61B 90/00

(54) **GUIDEWIRE CATHETER DELIVERING DEVICE**
FÜHRUNGSDRAHTKATHETER-ABGABEVORRICHTUNG
DISPOSITIF D'ADMINISTRATION DE CATHÉTER À FIL-GUIDE

(30) Priority: 23.02.2022 CN 202210169988
(43) Date of publication of application: 25.12.2024
(73) Proprietor: Shanghai Operation Robot Co., Ltd., Shanghai 201318 (CN)
(72) Inventor: LIU, Wenming, Shanghai 201318 (CN); YU, Zhongwei, Shanghai 201318 (CN); LIU, Daozhi, Shanghai 201318 (CN); LIU, Yikun, Shanghai 201318 (CN)
(74) Representative: Novagraaf Group
(86) International application number: PCT/CN2022/140594
(87) International publication number: WO 2023/160185

(56) References cited:
- WO-A1-2021/015990
- CN-A- 101 791 233
- CN-A- 105 596 084
- CN-A- 105 664 333
- CN-A- 108 309 370
- CN-A- 110 624 171
- CN-A- 111 202 586
- CN-A- 112 137 723
- CN-A- 113 729 966
- CN-A- 113 995 941
- CN-A- 114 587 616
- JP-A- 2017 023 212
- US-A1- 2010 292 566
- US-A1- 2020 338 287
- US-B2- 10 903 725

## Description

### TECHNICAL FIELD

The present invention relates to the field of medical devices and particularly to a guidewire catheter delivery device.

### BACKGROUND TECHNOLOGY

As a very mature endoscopic minimally invasive technique in the treatment of diseases of the biliary pancreatic system, endoscopic retrograde cholangiopancreatography (ERCP) can be used to diagnose and treat gallstones, biliary obstruction, cholangitis, biliary tumors, pancreatic tumors, and the like. During the operation, a duodenoscope is inserted into the descending part of the duodenum of the patient, an angiographic catheter is inserted into the biopsy tube to reach the duodenal papilla opening, and then contrast agent is injected. The specific pancreaticobiliary conditions are observed under the X-ray film to determine whether there were lesions, and then the corresponding operation is carried out. ERCP has the advantages of less trauma, short operation time, few complications, and high safety. ERCP, as minimally invasive surgery, just causes a small surgical trauma, does not bring too much pain to the patient, and has fast postoperative recovery, but it needs to be done with the aid of X-rays, and the doctor for this surgery must be exposed to X-rays for a long time. Therefore, in order to improve the working environment of the doctors for interventional surgery, ERCP surgical robots have been developed in engineering to substitute doctors and complete surgery. As a result, doctors control ERCP surgical robots to complete surgery in an X-ray free environment through remote operation or remote control. At present, there is no effective and similar technical solution on the market.

At present, ERCP operations in China are performed manually by doctors and their teams. During the operation, the operators need to wear heavy anti-radiation suits, but the arm part must be exposed for operation, which cannot be protected against radiation. Long-term surgical radiation can cause serious radiation damage to the operators. In addition, the existing ERCP operation requires a lot of operators and cooperators, so it is crowded in the space-limited operating room. Doctors and operators need to stand all day long for surgery, which causes high-intensity work, and it is easy to fatigue and thus affect the accuracy of surgery and even lead to mistakes. Additionally, during the operation, it is difficult for the doctors and the operators to ensure that the hand does not shake, so the displacement may occur after the inserted human instrument is positioned.

The patent document (CN105664333A) discloses a guidewire catheter delivery device, including a guidewire, a catheter, a guidewire delivery system, and a catheter delivery system. The catheter delivery system includes a catheter delivery guide rail and can move along the axial direction of the catheter delivery guide rail, and the catheter is fixed on the catheter delivery system. The guidewire delivery system includes a guidewire delivery guide rail and can move along the axial direction of the guidewire delivery guide rail, and the guidewire is fixed on the guidewire delivery system. The guidewire delivery guide rail is fixed on the catheter delivery guide rail, and the guidewire is coaxial with the catheter.

The patent document (CN110624171A) discloses a guidewire catheter delivery device, including a guidewire moving clamping mechanism, a guidewire fixing clamping mechanism, a guidewire axial moving mechanism, a catheter delivery clamping mechanism, and a base. The guidewire moving clamping mechanism, the guidewire fixing clamping mechanism, the guidewire axial moving mechanism and the catheter delivery clamping mechanism are respectively provided on the base along the axial feeding direction of the guidewire.

CN112137723A discloses an interventional robot. The robot comprises a master end, a slave end and a control device connected with the master end and the slave end, wherein the master end comprises an operation device, an operation detection device and a resistance generation device; the operation device is in transmission connection with the resistance generation device through a one-way transmission mechanism; the operation detection device is connected with the control device; the control device collects a measurement signal of the operation detection device to control the slave end; the slave end comprises a fixed platform; a guide rail and a guide rail driving mechanism corresponding to the guide rail are arranged on the fixed platform; a conveying mechanism is arranged on the guide rail; the guide rail driving mechanism drives the conveying mechanism to slide along the guide rail; the slave end further comprises a resistance detection device connected with the control device; and the control device collects a measurement signal of the resistance detection device to control the resistance generation device. US2010/292566A1 discloses load detection device and load detection method. According to this load detection method, load (Fi) in insertion of a linear body having flexibility in an inlet of a hollow tube inserted in a bending vessel in a body is detected, an image of the hollow tube or the linear body is taken, a degree of bending of the hollow tube or the linear body is detected based on the image, and load (Fo) at a tip end of the linear body is calculated based on the detected insertion load (Fi) and the degree of bending. Therefore, the load (Fo) at the tip end of the thin linear body can readily be detected. US10903725B2 discloses a compact height torque sensing articulation axis assembly having a torque sensor, an assembly mounting flange, a motor, a motor gearbox, a gearbox output shaft, an encoder, and a cable. The assembly may sense tension on robotic catheter pull wires in an articulating catheter and/or torque on a robotic output axis using the torque sensor. WO2021/015990A1 discloses an apparatus, wherein the apparatus includes a drive module having a drive module base component and a load-sensed component. An elongated medical device (EMD) is removably coupled to an isolated component. The isolated component is isolated from an external load other than an actual load acting on the EMD. The isolated component is removably coupled to the load-sensed component. A load sensor is secured to the drive module base component and the load-sensed component sensing the actual load acting on the EMD.

### CONTENT OF THE INVENTION

Aiming at the shortcomings in the prior art, the object of the present invention is to provide a guidewire catheter delivery device.

The invention is defined by the independent claim.

According to one or more embodiments of the present invention, a guidewire catheter delivery device includes: a guidewire catheter delivery module and a motor drive and force feedback module;
the motor drive and force feedback module is drivingly connected to the guidewire catheter delivery module, and the guidewire catheter delivery module is configured to be installed with a guidewire catheter and configured to drive the guidewire catheter to move back and forth;
a force sensor and a motor are installed in the motor drive and force feedback module;
an output end of the motor is connected to the guidewire catheter delivery module, and an end, facing away from the guidewire catheter delivery module, of the motor is connected to the force sensor;
a driving wheel and a driven wheel are installed in the guidewire catheter delivery module; and
the driving wheel is connected to the output end of the motor, and the guidewire catheter is configured to be installed between the driving wheel and the driven wheel;
wherein the motor drive and force feedback module further comprises: a motor support frame and a support shaft;
the force sensor and the motor are installed in the motor support frame, the output end of the motor extends out of one end of the motor support frame, and the force sensor is installed at the other end of the motor support frame; and
the support shaft extends outward from a middle of the motor support frame;
wherein the motor support frame is installed in a fixed seat;
the fixed seat is fixedly connected to the force sensor;
wherein the motor support frame is rotatably connected to the fixed seat through the support shaft, such that motor support frame is configured to swing about a rotation axis of the support shaft, wherein the rotation axis of the support shaft is perpendicular to a rotation axis of the output end of the motor;
wherein when the guidewire catheter moves forward and backward, the motor receives a reaction force from the guidewire catheter, the end, adjacent to the guidewire catheter delivery module, of the motor support frame receives the reaction force, and the end, connected to the force sensor, of the motor support frame receives a force that is opposite to the reaction force through the support shaft, thereby causing the motor support frame to swing for sequentially generating a pulling force or pressing force on the force sensor,
wherein the force sensor includes one end connected to the motor support frame, and the other end connected to the fixed seat to ensure that the pulling force or pressing force is generated.

Further, preferably, the guidewire catheter delivery module further includes: a main body, a flip cover, a driven wheel frame, and a bottom cover;
the bottom cover is installed at a bottom of the main body, and the flip cover is rotatably connected to the main body through a shaft pin; the main body, the bottom cover, and the flip cover are surrounded to form a cavity;
the driven wheel frame, the driving wheel, and the driven wheel are installed in the cavity;
the driving wheel is installed on a side of the driven wheel frame, and the driven wheel is installed inside the driven wheel frame;
the driven wheel frame is allowed to drive the driven wheel to move relative to the driving wheel and the main body;
when the flip cover rotates away from the main body through the shaft pin to be opened, the guidewire catheter is allowed to be installed in the main body; and
when the flip cover rotates towards the main body through the shaft pin to be closed, the flip cover is configured to limit movement of the guidewire catheter.

Further, preferably, the driven wheel is rotatably connected to the driven wheel frame, and the driven wheel is allowed to move towards or away from the driving wheel relative to the driven wheel frame;
a driven wheel pressing ball is installed on a side, facing away from the driving wheel, of the driven wheel frame, and a driven wheel pressing spring is installed between the driven wheel and the driven wheel pressing ball; and
when the flip cover is closed, the driven wheel frame is pressed by the flip cover to move towards the driving wheel, and the driven wheel pressing spring presses the driven wheel towards the driving wheel.

Further, preferably, a limit block is installed on a side wall of the driven wheel frame; and
when the driven wheel frame moves away from the driving wheel, the limit block interferes with the main body, and the driven wheel frame limits a distance away from the driving wheel through the limit block.

Further, preferably, a boss is provided on a side, adjacent to the driving wheel, of an inner wall of the main body, and a driven wheel release spring is installed between the limit block and the boss; and
when the flip cover is opened, the driven wheel frame moves away from the driving wheel through the driven wheel release spring.

Further, preferably, buckles are provided on sides, facing the motor drive and force feedback module, of the flip cover and the bottom cover; and
the buckles are clamped on the motor support frame.

Further, preferably, the driven wheel is installed on the driven wheel frame by a screw and a nut;
the driven wheel is allowed to rotate around the screw;
the screw and the nut are allowed to move relative to the main body; and
a first bearing and a second bearing are installed between the driving wheel and the main body, and a third bearing is installed between the driven wheel and the screw.

The present invention further provides a robot including the guidewire catheter delivery device.

### DESCRIPTION OF THE DRAWINGS

Other features, objects, and advantages of the present invention will become more apparent by reading the detailed description of non-limiting embodiments with reference to the following drawings:
FIG. 1 is an exploded view of a guidewire catheter delivery device;
FIG. 2 is a schematic diagram showing the structure of the guidewire catheter delivery device;
FIG. 3 is a front view of the guidewire catheter delivery device;
FIG. 4 is a schematic diagram showing the structure of a guidewire catheter delivery module;
FIG. 5 is a cross-section view of the guidewire catheter delivery module;
FIG. 6 is a cross-section view of the guidewire catheter delivery device;
FIG. 7 is a schematic diagram showing the structure of the guidewire catheter delivery module after the flip cover is opened;
FIG. 8 is a cross-section view of a motor drive and force feedback module;
FIG. 9 is a schematic diagram showing the structure of a screw; and
FIG. 10 is a schematic diagram showing the structure of a nut.

The reference numerals in the drawings are as follows:

| | | | |
|---|---|---|---|
| guidewire catheter delivery module | 1 | bottom cover | 113 |
| main body | 101 | limit block | 114 |
| flip cover | 102 | driven wheel pressing spring | 115 |
| driving wheel | 103 | | |
| driven wheel | 104 | motor drive and force feedback module | 2 |
| driven wheel frame | 105 | motor support frame | 201 |
| driven wheel release spring | 106 | force sensor | 202 |
| driven wheel pressing ball | 107 | motor | 203 |
| screw | 108 | support shaft | 204 |
| nut | 109 | fixed seat | 205 |
| first bearing | 110 | | |
| second bearing | 111 | guidewire catheter | 3 |
| third bearing | 112 | | |

### SPECIFIC IMPLEMENTATIONS

The present invention will be described in detail below in conjunction with specific embodiments. The following embodiments will help those skilled in the art to further understand the present invention, but do not limit the present invention in any form. It should be pointed out that for those having ordinary skill in the art, several changes and improvements may be made without departing from the ideas of the present invention. These all fall within the protection scope of the present invention.

### Embodiment 1

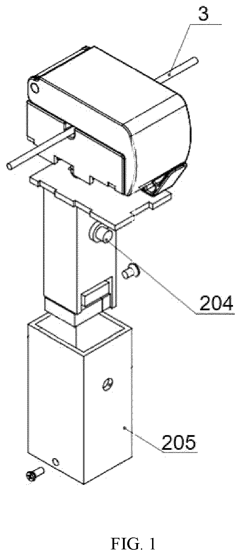

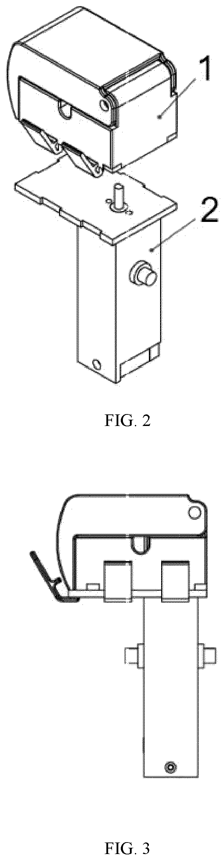

As shown in FIGS. 1 to 3, a guidewire catheter delivery device, which can be used for a robot, includes: the guidewire catheter delivery module 1 and the motor drive and force feedback module 2. The motor drive and force feedback module 2 is drivingly connected to the guidewire catheter delivery module 1, and the guidewire catheter delivery module 1 is installed with the guidewire catheter 3 and drives the guidewire catheter 3 to move back and forth. The force sensor 202 and the motor 203 are installed in the motor drive and force feedback module 2. An output end of the motor 203 is connected to the guidewire catheter delivery module 1, and an end, facing away from the guidewire catheter delivery module 1, of the motor 203 is connected to the force sensor 202. The driving wheel 103 and the driven wheel 104 are installed in the guidewire catheter delivery module 1. The driving wheel 103 is connected to the output end of the motor 203. The guidewire catheter 3 is installed between the driving wheel 103 and the driven wheel 104.

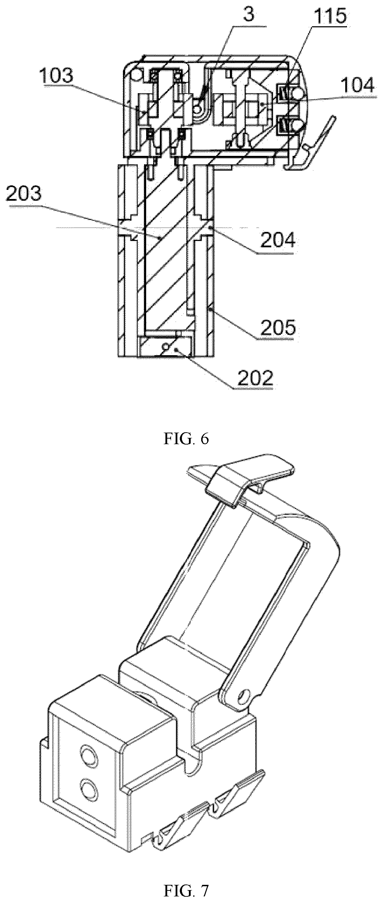

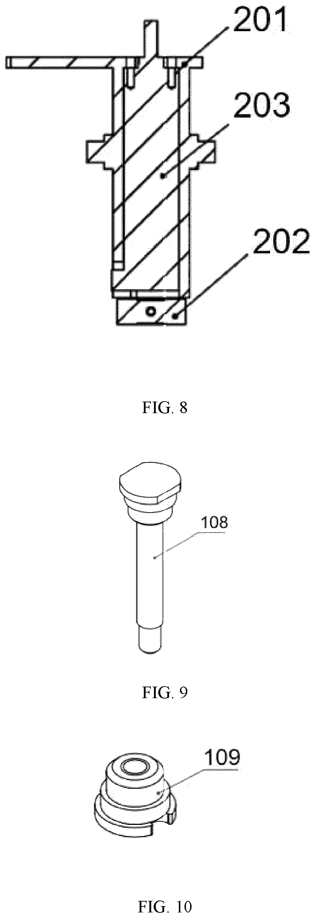

As shown in FIGS. 6 and 8, the motor drive and force feedback module 2 further includes: the motor support frame 201 and the support shaft 204. The force sensor 202 and the motor 203 are installed in the motor support frame 201. The output end of the motor 203 extends out of one end of the motor support frame 201, and the force sensor 202 is installed at the other end of the motor support frame 201. The support shaft 204 extends outward from a middle of the motor support frame 201. The motor support frame 201 is installed in the fixed seat 205, the motor support frame 201 is rotatably connected to the fixed seat 205 through the support shaft 204, and the fixed seat 205 is fixedly connected to the force sensor 202. When the guidewire catheter 3 moves, the motor 203 receives the reaction force from the guidewire catheter 3, the end, adjacent to the guidewire catheter delivery module 1, of the motor support frame 201 receives the reaction force, and the end, connected to the force sensor 202, of the motor support frame 201 receives the force that is opposite to the reaction force through the support shaft 204.

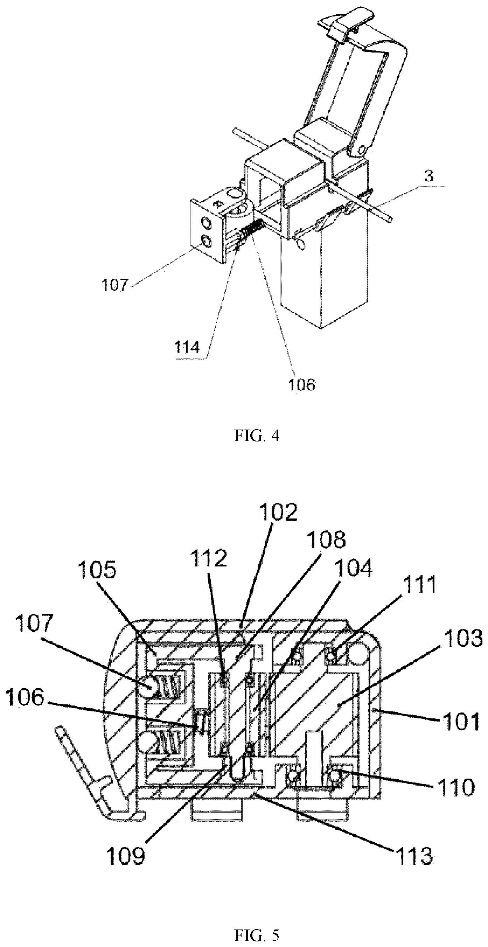

As shown in FIGS. 4 and 5, the guidewire catheter delivery module 1 further includes: the main body 101, the flip cover 102, the driven wheel frame 105, and the bottom cover 113. The bottom cover 113 is installed at the bottom of the main body 101, and the flip cover 102 is rotatably connected to the main body 101 through a shaft pin. The main body 101, the bottom cover 113, and the flip cover 102 are surrounded to form a cavity. The driven wheel frame 105, the driving wheel 103 and the driven wheel 104 are installed in the cavity. The driving wheel 103 is installed on a side of the driven wheel frame 105, and the driven wheel 104 is installed inside the driven wheel frame 105. The driven wheel frame 105 is allowed to drive the driven wheel 104 to move relative to the driving wheel 103 and the main body 101. When the flip cover 102 rotates away from the main body 101 through the shaft pin to be opened, the guidewire catheter 3 is allowed to be installed in the main body 101. When the flip cover 102 rotates towards the main body 101 through the shaft pin to be closed, the flip cover 102 limits the movement of the guidewire catheter 3. The driven wheel 104 is rotatably connected to the driven wheel frame 105, and the driven wheel 104 is allowed to move towards or away from the driving wheel 103 relative to the driven wheel frame 105. The driven wheel pressing ball 107 is installed on the side, facing away from the driving wheel 103, of the driven wheel frame 105, and the driven wheel pressing spring 115 is installed between the driven wheel 104 and the driven wheel pressing ball 107. When the flip cover 102 is closed, the driven wheel frame 105 is pressed by the flip cover 102 to move towards the driving wheel 103, and the driven wheel pressing spring 115 presses the driven wheel 104 towards the driving wheel 103. The limit block 114 is installed on a side wall of the driven wheel frame 105. When the driven wheel frame 105 moves away from the driving wheel 103, the limit block 114 interferes with the main body 101, and the driven wheel frame 105 limits the distance away from the driving wheel 103 through the limit block 114. A boss is provided on the side, adjacent to the driving wheel 103, of the inner wall of the main body 101, and the driven wheel release spring 106 is installed between the limit block 114 and the boss. When the flip cover 102 is opened, the driven wheel frame 105 moves away from the driving wheel 103 through the driven wheel release spring 106.

As shown in FIGS. 7, 9 and 10, buckles are provided on the sides, facing the motor drive and force feedback module 2, of the flip cover 102 and the bottom cover 113, and the buckles are clamped on the motor support frame 201. The driven wheel 104 is installed on the driven wheel frame 105 by the screw 108 and the nut 109, the driven wheel 104 is allowed to rotate around the screw 108, and the screw 108 and nut 109 are allowed to move relative to the main body 101. The first bearing 110 and the second bearing 111 are installed between the driving wheel 103 and the main body 101, and the third bearing 112 is installed between the driven wheel 104 and the screw 108.

### Embodiment 2

As shown in FIGS. 1 to 3, this embodiment includes: the guidewire catheter delivery module 1 and the motor drive and force feedback module 2. The guidewire catheter delivery module 1 is installed on the motor drive and force feedback module 2 and driven by the motor drive and force feedback module 2. The driving wheel 103 and the driven wheel 104 are installed in the guidewire catheter delivery module 1. The force sensor 202 and the motor 203 are installed in the motor drive and force feedback module 2, and an output end of the motor 203 is drivingly connected to the driving wheel 103. When the guidewire catheter 3 is pushed, the driving wheel 103 and the driven wheel 104 rotate to push the guidewire catheter 3 to move, and the guidewire catheter 3 generates a reverse force on the driving wheel 103 and the motor 203. The motor support frame 201 forms a lever through the support shaft 204, and the force sensor 202 generates a signal of a tensile force opposite to the force exerted on the motor 203.

As shown in FIGS. 4 and 5, the guidewire catheter delivery module 1 includes: the main body 101, the flip cover 102, the driving wheel 103, the driven wheel 104, the driven wheel frame 105, the driven wheel release spring 106, the driven wheel pressing ball 107, the screw 108, the nut 109, the first bearing 110, the second bearing 111, the third bearing 112, and the bottom cover 13. After the main body 101 and the flip cover 102 are connected through a shaft pin, the flip cover 102 can be opened to facilitate the placement or removement of the guidewire catheter 3. The driving wheel 103 is fixed on the main body 101 through the first bearing 110 and the second bearing 111, and the driven wheel 104 is fixed on the driven wheel frame 105 through the third bearing 112, the screw 108, and the nut 109.

As shown in FIGS. 6 to 10, the driven wheel release spring 106 and the driven wheel pressing ball 107 are fixed on the driven wheel frame 105. The driven wheel pressing ball 107 is installed on the side, facing away from the driving wheel 103, of the driven wheel frame 105. The driven wheel pressing spring 115 is installed between the driven wheel 104 and the driven wheel pressing ball 107. A boss is provided on the side, adjacent to the driving wheel 103, of the inner wall of the main body 101, the driven wheel release spring 106 is installed between the limit block 114 and the boss, and the limit block 114 is installed on the side wall of the driven wheel frame 105. When the driven wheel frame 105 moves away from the driving wheel 103, the limit block 114 interferes with the main body 101, the driven wheel frame 105 limits the distance away from the driving wheel 103 through the limit block 114, and the driven wheel release spring 106 and the driven wheel pressing spring 115 can control the driven wheel 104 to tightly press or release the guidewire catheter 3. The bottom cover 113 is fixedly connected to the main body 101.

The force sensor 202 and the motor 203 are fixed on the motor support frame 201. Two support shafts 204 are provided on the side of the motor support frame 201 and are rotatably installed on the fixed seat 205. The guidewire catheter delivery module 1 is fixed on the motor drive and force feedback module 2 through a buckle, and the entire guidewire catheter delivery module 1 can be released by pressing the buckle on one side. The flip cover 102 can also be fixed on the motor drive and force feedback module 2 through a buckle, and can also be released by a button. The D-shaped shaft of the motor 203 is matched with the D-shaped hole in the driving wheel 103, and after connection, the motor 203 can drive the driving wheel 103 to rotate. The flip cover 102 can be opened and locked at any time, and can be used for positioning, loading and unloading the guidewire catheter 3 at the same time. When the flip cover 102 is closed, the upward and downward movement of the guidewire catheter 3 is limited. When the flip cover 102 is opened, the guidewire catheter 3 can be put in or taken out from above. When the flip cover 102 is opened, the internal driven wheel release spring 106 pushes out the driven wheel 104, and the guidewire catheter 3 is released. When the flip cover 102 is closed, the external driven wheel pressing spring 115 presses the driven wheel 104 tightly, so that the guidewire catheter 3 is clamped tightly, and finally the motor 203 can drive the advancement or retraction of the guidewire catheter 3. The driven wheel frame 105 and the driven wheel 104 are assembled by the screw 108 and the nut 109 to translate within the main body 101. When the guidewire catheter 3 moves forward and backward, the motor support frame 201 tends to swing, which sequentially generates a pulling force or pressing force on the force sensor 202 installed at the bottom. The force sensor 202 at the bottom includes one end connected to the motor support frame 201, and the other end connected to the fixed seat 205 to ensure that the pulling force or pressing force can be generated.

Those skilled in the art should know that, in addition to realizing the system and its various devices, modules and units provided by the present invention in a purely computer-readable program code form, through logical programming of the method steps, the system and its various devices, modules and units provided by the present invention can realize the same functions in the form of logic gates, switches, application-specific integrated circuits, programmable logic controllers, embedded microcontrollers, etc. Therefore, the system and its various devices, modules and units provided by the present invention can be regarded as a hardware component, and the devices, modules and units included therein for realizing various functions can also be regarded as structures within the hardware component; or the devices, modules and units for realizing various functions can also be regarded as either software modules for implementing methods or structures within hardware components.

In the description of the present application, it should be understood that the orientations or positional relationships indicated by the terms "upper", "lower", "front", "rear", "left", "right", "vertical", "horizontal", "top", "bottom", "inner", "outer", etc. are based on the orientations or positional relationships shown in the drawings, are only for convenience in describing the present application and simplifying the description, and do not indicate or imply that the devices or elements referred to must have a specific orientation or be constructed and operated in a specific orientation. Therefore, they cannot be construed as a limitation on the present application.

Compared with the prior art, the present invention has the following beneficial effects:
1. The present invention is practical since it can be used in a surgical robot for intervention to avoid surgical radiation and improve surgical safety;
2. The present invention realizes the delivery of the guidewire catheter and has high stability;
3. The device provided by the present invention has a compact structure and saves space.

## Claims

1. A guidewire catheter delivery device, comprising a guidewire catheter delivery module (1) and a motor drive and force feedback module (2);
the motor drive and force feedback module (2) is drivingly connected to the guidewire catheter delivery module (1), and the guidewire catheter delivery module (1) is configured to be installed with a guidewire catheter (3) and configured to drive the guidewire catheter (3) to move back and forth;
a force sensor (202) and a motor (203) are installed in the motor drive and force feedback module (2);
an output end of the motor (203) is connected to the guidewire catheter delivery module (1), and an end, facing away from the guidewire catheter delivery module (1), of the motor (203) is connected to the force sensor (202);
a driving wheel (103) and a driven wheel (104) are installed in the guidewire catheter delivery module (1); and
the driving wheel (103) is connected to the output end of the motor (203), and the guidewire catheter (3) is configured to be
installed between the driving wheel (103) and the driven wheel (104);
wherein the motor drive and force feedback module (2) further comprises: a motor support frame (201) and a support shaft (204);
the force sensor (202) and the motor (203) are installed in the motor support frame (201), the output end of the motor (203) extends out of one end of the motor support frame (201), and the force sensor (202) is installed at the other end of the motor support frame (201); and
the support shaft (204) extends outward from a middle of the motor support frame (201);
wherein the motor support frame (201) is installed in a fixed seat (205);
the fixed seat (205) is fixedly connected to the force sensor (202);
wherein the motor support frame (201) is rotatably connected to the fixed seat (205) through the support shaft (204), such that motor support frame (201) is configured to swing about a rotation axis of the support shaft (204), wherein the rotation axis of the support shaft (204) is perpendicular to a rotation axis of the output end of the motor (203);
wherein when the guidewire catheter (3) moves forward and backward, the motor (203) receives a reaction force from the guidewire catheter (3), the end, adjacent to the guidewire catheter delivery module (1), of the motor support frame (201) receives the reaction force, and the end, connected to the force sensor (202), of the motor support frame (201) receives a force that is opposite to the reaction force through the support shaft (204), thereby causing the motor support frame (201) to swing for sequentially generating a pulling force or pressing force on the force sensor (202),
wherein the force sensor (202) includes one end connected to the motor support frame (201), and the other end connected to the fixed seat (205) to ensure that the pulling force or pressing force is generated.

2. The guidewire catheter delivery device according to claim 1, wherein the guidewire catheter delivery module (1) further comprises a main body (101), a flip cover (102), a driven wheel frame (105), and a bottom cover (113);
the bottom cover (113) is installed at a bottom of the main body (101), and the flip cover (102) is rotatably connected to the main body (101) through a shaft pin; the main body (101), the bottom cover (113), and the flip cover (102) are surrounded to form a cavity;
the driven wheel frame (105), the driving wheel (103), and the driven wheel (104) are installed in the cavity;
the driving wheel (103) is installed on a side of the driven wheel frame (105), and the driven wheel (104) is installed inside the driven wheel frame (105);
the driven wheel frame (105) is allowed to drive the driven wheel (104) to move relative to the driving wheel (103) and the main body (101);
when the flip cover (102) rotates away from the main body (101) through the shaft pin to be opened, the guidewire catheter (3) is allowed to be installed in the main body (101); and
when the flip cover (102) rotates towards the main body (101) through the shaft pin to be closed, the flip cover (102) is configured to limit movement of the guidewire catheter (3).

3. The guidewire catheter delivery device according to claim 2, wherein the driven wheel (104) is rotatably connected to the driven wheel frame (105), and the driven wheel (104) is allowed to move towards or away from the driving wheel (103) relative to the driven wheel frame (105);
a driven wheel pressing ball (107) is installed on a side, facing away from the driving wheel (103), of the driven wheel frame (105), and a driven wheel pressing spring (115) is installed between the driven wheel (104) and the driven wheel pressing ball (107); and
when the flip cover (102) is closed, the driven wheel frame (105) is pressed by the flip cover (102) to move towards the driving wheel (103), and the driven wheel pressing spring (115) presses the driven wheel (104) towards the driving wheel (103).

4. The guidewire catheter delivery device according to claim 2, wherein a limit block (114) is installed on a side wall of the driven wheel frame (105); and
when the driven wheel frame (105) moves away from the driving wheel (103), the limit block (114) interferes with the main body (101), and the driven wheel frame (105) limits a distance away from the driving wheel (103) through the limit block (114).

5. The guidewire catheter delivery device according to claim 4, wherein a boss is provided on a side, adjacent to the driving wheel (103), of an inner wall of the main body (101), and a driven wheel release spring (106) is installed between the limit block (114) and the boss; and
when the flip cover (102) is opened, the driven wheel frame (105) moves away from the driving wheel (103) through the driven wheel release spring (106).

6. The guidewire catheter delivery device according to claim 2, wherein buckles are provided on sides, facing the motor drive and force feedback module (2), of the flip cover (102) and the bottom cover (113); and
the buckles are clamped on the motor support frame (201).

7. The guidewire catheter delivery device according to claim 2, wherein the driven wheel (104) is installed on the driven wheel frame (105) by a screw (108) and a nut (109);
the driven wheel (104) is allowed to rotate around the screw (108);
the screw (108) and the nut (109) are allowed to move relative to the main body (101); and
a first bearing (110) and a second bearing (111) are installed between the driving wheel (103) and the main body (101), and a third bearing (112) is installed between the driven wheel (104) and the screw (108).

8. A robot, comprising the guidewire catheter delivery device according to any one of claims 1 to 7.

## Patentansprüche

1. Führungsdrahtkatheterabgabevorrichtung, umfassend ein Führungsdrahtkatheterabgabemodul (1) und ein Motorantriebs- und Kraftrückkopplungsmodul (2);
wobei das Motorantriebs- und Kraftrückkopplungsmodul (2) mit dem Führungsdrahtkatheterabgabemodul (1) antriebsverbunden ist, und das Führungsdrahtkatheterabgabemodul (1) konfiguriert ist, um mit einem Führungsdrahtkatheter (3) installiert zu werden und konfiguriert ist, um den Führungsdrahtkatheter (3) anzutreiben, um sich hin und her zu bewegen;
ein Kraftsensor (202) und ein Motor (203) in dem Motorantriebs- und Kraftrückkopplungsmodul (2) installiert sind;
eine Abtriebsseite des Motors (203) mit dem
Führungsdrahtkatheterabgabemodul (1) verbunden ist und ein Ende des Motors (203), das von dem Führungsdrahtkatheterabgabemodul (1) abgewandt ist, mit dem Kraftsensor (202) verbunden ist;
ein Antriebsrad (103) und ein angetriebenes Rad (104) in dem Führungsdrahtkatheterabgabemodul (1) installiert sind; und
das Antriebsrad (103) mit der Abtriebsseite des Motors (203) verbunden ist und der Führungsdrahtkatheter (3) konfiguriert ist, um zwischen dem Antriebsrad (103) und dem angetriebenen Rad (104) installiert zu werden;
wobei das Motorantriebs- und Kraftrückkopplungsmodul (2) ferner umfasst: einen Motorstützrahmen (201) und eine Stützwelle (204);
der Kraftsensor (202) und der Motor (203) in dem Motorstützrahmen (201) installiert sind, sich die Abtriebsseite des Motors (203) aus einem Ende des Motorstützrahmens (201) heraus erstreckt und der Kraftsensor (202) an dem anderen Ende des Motorstützrahmens (201) installiert ist; und
sich die Stützwelle (204) von einer Mitte des Motorstützrahmens (201) nach außen erstreckt;
der Motorstützrahmen (201) in einem festen Sitz (205) installiert ist;
der feste Sitz (205) mit dem Kraftsensor (202) fest verbunden ist;
wobei der Motorstützrahmen (201) über die Stützwelle (204) mit dem festen Sitz (205) derart drehbar verbunden ist, dass der Motorstützrahmen (201) konfiguriert ist, um um eine Drehachse der Stützwelle (204) herum zu schwingen, wobei die Drehachse der Stützwelle (204) senkrecht zu einer Drehachse der Abtriebsseite des Motors (203) ist;
wobei, wenn sich der Führungsdrahtkatheter (3) hin und her bewegt, der Motor (203) eine Reaktionskraft von dem Führungsdrahtkatheter (3) empfängt, das Ende des Motorstützrahmens (201), das an das Führungsdrahtkatheterabgabemodul (1) angrenzt, die Reaktionskraft empfängt, und das Ende des Motorstützrahmens (201), das mit dem Kraftsensor (202) verbunden ist, eine Kraft empfängt, die der Reaktionskraft über die Stützwelle (204) entgegengesetzt ist, wodurch bewirkt wird. dass der Motorstützrahmen (201) zum sequenziellen Erzeugen einer Zugkraft oder Presskraft auf den Kraftsensor (202) schwingt,
wobei der Kraftsensor (202) ein Ende einschließt, das mit dem Motorstützrahmen (201) verbunden ist, und das andere Ende mit dem festen Sitz (205) verbunden ist, um sicherzustellen, dass die Zugkraft oder Presskraft erzeugt wird.

2. Führungsdrahtkatheterabgabevorrichtung nach Anspruch 1, wobei das Führungsdrahtkatheterabgabemodul (1) ferner einen Hauptkörper (101), eine Klappabdeckung (102), einen Rahmen (105) des angetriebenen Rades und eine Bodenabdeckung (113) umfasst;
die untere Abdeckung (113) an einer Unterseite des Hauptkörpers (101) installiert ist und die Klappabdeckung (102) über einen Wellenstift mit dem Hauptkörper (101) drehbar verbunden ist; der Hauptkörper (101), die Bodenabdeckung (113) und die Klappabdeckung (102) umgeben sind, um einen Hohlraum auszubilden;
der Rahmen (105) des angetriebenen Rades, das Antriebsrad (103) und das Antriebsrad (104) in dem Hohlraum installiert sind;
das Antriebsrad (103) an einer Seite des Rahmens (105) des angetriebenen Rades installiert ist und das Antriebsrad (104) innerhalb des Rahmens (105) des angetriebenen Rades installiert ist;
es dem Rahmen (105) des angetriebenen Rades erlaubt ist, das Antriebsrad (104) anzutreiben, um sich relativ zu dem Antriebsrad (103) und dem Hauptkörper (101) zu bewegen;
wenn sich die Klappabdeckung (102) über den Wellenstift von dem Hauptkörper (101) weg dreht, um geöffnet zu werden, es dem der Führungsdrahtkatheter (3) erlaubt ist, in dem Hauptkörper (101) installiert zu werden; und
wenn sich die Klappabdeckung (102) über den Wellenstift in Richtung des Hauptkörpers (101) dreht, um geschlossen zu werden, die Klappabdeckung (102) konfiguriert ist, um eine Bewegung des Führungsdrahtkatheters (3) zu begrenzen.

3. Führungsdrahtkatheterabgabevorrichtung nach Anspruch 2, wobei das Antriebsrad (104) mit dem Rahmen (105) des angetriebenen Rades drehbar verbunden ist und es dem Antriebsrad (104) erlaubt ist, sich relativ zu dem Rahmen (105) des angetriebenen Rades auf das Antriebsrad (103) zu oder davon weg zu bewegen;
eine Antriebsradpresskugel (107) auf einer Seite des Rahmens (105) des angetriebenen Rades installiert ist, die von dem Antriebsrad (103) abgewandt ist, und eine Antriebsradpressfeder (115) zwischen dem Antriebsrad (104) und der Antriebsradpresskugel (107) installiert ist; und
wenn die Klappabdeckung (102) geschlossen ist, der Rahmen (105) des angetriebenen Rades durch die Klappabdeckung (102) gepresst wird, um sich in Richtung des Antriebsrades (103) zu bewegen, und die Antriebsradpressfeder (115) das Antriebsrad (104) in Richtung des Antriebsrades (103) presst.

4. Führungsdrahtkatheterabgabevorrichtung nach Anspruch 2, wobei ein Begrenzungsblock (114) an einer Seitenwand des Rahmens (105) des angetriebenen Rades installiert ist; und
wenn sich der Rahmen (105) des angetriebenen Rades von dem Antriebsrad (103) weg bewegt, der Begrenzungsblock (114) mit dem Hauptkörper (101) interferiert und der Rahmen (105) des angetriebenen Rades einen Abstand von dem Antriebsrad (103) weg über den Begrenzungsblock (114) begrenzt.

5. Führungsdrahtkatheterabgabevorrichtung nach Anspruch 4, wobei ein Vorsprung an einer Seite einer Innenwand des Hauptkörpers (101), die an das Antriebsrad (103) angrenzt, bereitgestellt ist und eine Antriebsradauslösefeder (106) zwischen dem Begrenzungsblock (114) und dem Vorsprung installiert ist; und
wenn die Klappabdeckung (102) geöffnet wird, sich der Rahmen (105) des angetriebenen Rades über die Antriebsradauslösefeder (106) von dem Antriebsrad (103) weg bewegt.

6. Führungsdrahtkatheterabgabevorrichtung nach Anspruch 2, wobei Schnallen auf Seiten, die dem Motorantriebs- und Kraftrückkopplungsmodul (2) zugewandt sind, der Klappabdeckung (102) und der Bodenabdeckung (113) bereitgestellt sind; und
die Schnallen an dem Motorstützrahmen (201) festgeklemmt sind.

7. Führungsdrahtkatheterabgabevorrichtung nach Anspruch 2, wobei das Antriebsrad (104) an dem Rahmen (105) des angetriebenen Rades durch eine Schraube (108) und eine Mutter (109) installiert ist;
es dem Antriebsrad (104) erlaubt ist, sich um die Schraube (108) zu drehen;
es der Schraube (108) und der Mutter (109) erlaubt ist, sich relativ zu dem Hauptkörper (101) zu bewegen; und
ein erstes Lager (110) und ein zweites Lager (111) zwischen dem Antriebsrad (103) und dem Hauptkörper (101) installiert sind und ein drittes Lager (112) zwischen dem angetriebenen Rad (104) und der Schraube (108) installiert ist.

8. Roboter, umfassend die Führungsdrahtkatheterabgabevorrichtung nach einem der Ansprüche 1 bis 7.

## Revendications

1. Dispositif de pose de cathéter fil-guide, comprenant un module de pose de cathéter fil-guide (1) et un module d'entraînement motorisé et de retour de force (2) ;
le module d'entraînement motorisé et de retour de force (2) est relié par entraînement au module de pose de cathéter fil-guide (1), et le module de pose de cathéter fil-guide (1) est conçu pour être installé avec un cathéter fil-guide (3) et conçu pour entraîner le cathéter fil-guide (3) à se déplacer d'avant en arrière ;
un capteur de force (202) et un moteur (203) sont installés dans le module d'entraînement motorisé et de retour de force (2) ;
une extrémité de sortie du moteur (203) est reliée au module de pose de cathéter fil-guide (1), et une extrémité, opposée au module de pose de cathéter fil-guide (1), du moteur (203) est reliée au capteur de force (202) ;
une roue motrice (103) et une roue entraînée (104) sont installées dans le module de pose de cathéter fil-guide (1) ; et
la roue motrice (103) est reliée à l'extrémité de sortie du moteur (203), et le cathéter fil-guide (3) est conçu pour être installé entre la roue motrice (103) et la roue entraînée (104) ;
dans lequel le module d'entraînement motorisé et de retour de force (2) comprend en outre : un cadre de support de moteur (201) et un arbre de support (204) ;
le capteur de force (202) et le moteur (203) sont installés dans le cadre de support de moteur (201), l'extrémité de sortie du moteur (203) s'étend hors d'une extrémité du cadre de support de moteur (201), et le capteur de force (202) est installé au niveau de l'autre extrémité du cadre de support de moteur (201) ; et
l'arbre de support (204) s'étend vers l'extérieur à partir du milieu du cadre de support de moteur (201) ;
dans lequel le cadre de support de moteur (201) est installé dans un siège fixe (205) ;
le siège fixe (205) est relié à demeure au capteur de force (202) ;
dans lequel le cadre de support de moteur (201) est relié de manière rotative au siège fixe (205) par l'intermédiaire de l'arbre de support (204), de telle sorte que le cadre de support de moteur (201) est conçu pour osciller autour d'un axe de rotation de l'arbre de support (204), dans lequel l'axe de rotation de l'arbre de support (204) est perpendiculaire à un axe de rotation de l'extrémité de sortie du moteur (203) ;
dans lequel, lorsque le cathéter fil-guide (3) se déplace vers l'avant et vers l'arrière, le moteur (203) reçoit une force de réaction provenant du cathéter fil-guide (3), l'extrémité, adjacente au module de pose de cathéter fil-guide (1), du cadre de support de moteur (201) reçoit la force de réaction, et l'extrémité, reliée au capteur de force (202), du cadre de support de moteur (201) reçoit une force qui est opposée à la force de réaction à travers l'arbre de support (204), amenant de ce fait le cadre de support de moteur (201) à osciller pour générer séquentiellement une force de traction ou une force de pression sur le capteur de force (202),
dans lequel le capteur de force (202) comporte une extrémité reliée au cadre de support de moteur (201), et l'autre extrémité reliée au siège fixe (205) pour garantir que la force de traction ou la force de pression est générée.

2. Dispositif de pose de cathéter fil-guide selon la revendication 1, dans lequel le module de pose de cathéter fil-guide (1) comprend en outre un corps principal (101), un couvercle rabattable (102), un cadre de roue entraînée (105) et un couvercle inférieur (113) ;
le couvercle inférieur (113) est installé au niveau d'une partie inférieure du corps principal (101), et le couvercle rabattable (102) est relié de manière rotative au corps principal (101) par l'intermédiaire d'une goupille d'arbre ; le corps principal (101), le couvercle inférieur (113) et le couvercle rabattable (102) sont entourés pour former une cavité ;
le cadre de roue entraînée (105), la roue motrice (103) et la roue entraînée (104) sont installés dans la cavité ;
la roue motrice (103) est installée sur un côté du cadre de roue entraînée (105), et la roue entraînée (104) est installée à l'intérieur du cadre de roue entraînée (105) ;
le cadre de roue entraînée (105) est autorisé à entraîner la roue entraînée (104) à se déplacer par rapport à la roue motrice (103) et au corps principal (101) ;
lorsque le couvercle rabattable (102) tourne en s'éloignant du corps principal (101) à travers la goupille d'arbre pour être ouvert, le cathéter fil-guide (3) peut être installé dans le corps principal (101) ; et
lorsque le couvercle rabattable (102) tourne vers le corps principal (101) à travers la goupille d'arbre pour être fermé, le couvercle rabattable (102) est conçu pour limiter un mouvement du cathéter fil-guide (3).

3. Dispositif de pose de cathéter fil-guide selon la revendication 2, dans lequel la roue entraînée (104) est reliée de manière rotative au cadre de roue entraînée (105), et la roue entraînée (104) peut se rapprocher ou s'éloigner de la roue motrice (103) par rapport au cadre de roue entraînée (105) ;
une bille de pressage de roue entraînée (107) est installée sur un côté, opposé à la roue motrice (103), du cadre de roue entraînée (105), et un ressort de pressage de roue entraînée (115) est installé entre la roue entraînée (104) et la bille de pressage de roue entraînée (107) ; et
lorsque le couvercle rabattable (102) est fermé, le cadre de roue entraînée (105) est pressé par le couvercle rabattable (102) pour se déplacer vers la roue motrice (103), et le ressort de pressage de roue entraînée (115) presse la roue entraînée (104) vers la roue motrice (103).

4. Dispositif de pose de cathéter fil-guide selon la revendication 2, dans lequel un bloc de butée (114) est installé sur une paroi latérale du cadre de roue entraînée (105) ; et
lorsque le cadre de roue entraînée (105) s'éloigne de la roue motrice (103), le bloc de butée (114) interfère avec le corps principal (101), et le cadre de roue entraînée (105) limite sa distance d'éloignement par rapport à la roue motrice (103) à travers le bloc de butée (114).

5. Dispositif de pose de cathéter fil-guide selon la revendication 4, dans lequel un bossage est prévu sur un côté, adjacent à la roue motrice (103), d'une paroi interne du corps principal (101), et un ressort de libération de roue entraînée (106) est installé entre le bloc de butée (114) et le bossage ; et
lorsque le couvercle rabattable (102) est ouvert, le cadre de roue entraînée (105) s'éloigne de la roue motrice (103) par l'intermédiaire du ressort de libération de roue entraînée (106).

6. Dispositif de pose de cathéter fil-guide selon la revendication 2, dans lequel des boucles sont prévues sur des côtés, faisant face vers le module d'entraînement motorisé et de retour de force (2), du couvercle rabattable (102) et du couvercle inférieur (113) ; et
les boucles sont serrées sur le cadre de support de moteur (201).

7. Dispositif de pose de cathéter fil-guide selon la revendication 2, dans lequel la roue entraînée (104) est installée sur le cadre de roue entraînée (105) par une vis (108) et un écrou (109) ;
la roue entraînée (104) peut tourner autour de la vis (108) ;
la vis (108) et l'écrou (109) peuvent se déplacer par rapport au corps principal (101) ; et
un premier roulement (110) et un deuxième roulement (111) sont installés entre la roue motrice (103) et le corps principal (101), et un troisième roulement (112) est installé entre la roue entraînée (104) et la vis (108).

8. Robot, comprenant le dispositif de pose de cathéter fil-guide selon l'une quelconque des revendications 1 à 7.
